(19) **Europäisches Patentamt** / **European Patent Office** / **Office européen des brevets**

(11) **EP 4 363 851 B1**

(12) **EUROPEAN PATENT SPECIFICATION**

(45) Date of publication and mention of the grant of the patent:
**19.11.2025 Bulletin 2025/47**

(21) Application number: **22741735.9**

(22) Date of filing: **30.06.2022**

(51) International Patent Classification (IPC):
***G01N 33/543*** $^{(2006.01)}$

(52) Cooperative Patent Classification (CPC):
**G01N 33/543**

(86) International application number:
**PCT/EP2022/068159**

(87) International publication number:
**WO 2023/275297 (05.01.2023 Gazette 2023/01)**

(54) **METHOD FOR CALIBRATING AN IMMUNOASSAY DEVICE TO DETERMINE THE CONCENTRATION OF AN ANALYTE**

VERFAHREN ZUM KALIBRIEREN EINER VORRICHTUNG ZUR BESTIMMUNG DER KONZENTRATION EINES ANALYTEN

PROCÉDÉ D'ÉTALONNAGE D'UN DISPOSITIF POUR DÉTERMINER LA CONCENTRATION D'UN ANALYTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2021 EP 21183194**

(43) Date of publication of application:
**08.05.2024 Bulletin 2024/19**

(73) Proprietors:
- **Radiometer Medical ApS**
  **2700 Brønshøj (DK)**
- **Radiometer Turku Oy**
  **20750 Turku (FI)**

(72) Inventors:
- **KAAE, Birgitte**
  **2700 Brønshøj (DK)**
- **COLDING-JØRGENSEN, Steffen**
  **2700 Brønshøj (DK)**
- **PORJO, Niko**
  **20750 Turku (FI)**

(74) Representative: **Maiwald GmbH**
**Engineering**
**Elisenhof**
**Elisenstrasse 3**
**80335 München (DE)**

(56) References cited:
**WO-A1-2014/143323    US-A- 3 960 497**
**US-B1- 6 277 584**

**Description**

The present invention relates to the field of molecular diagnostics, in particular to the calibration of an immunoassay device to determine the concentration of an analyte in a sample. The invention also relates to a method of determining the concentration of an analyte in a sample, where the concentration is measured with an immunoassay device that has been calibrated according to the calibration method of the present invention.

**BACKGROUND**

Generally, biochemical analysers employ a combination of analyte specific chemical reagents and reaction monitoring means to assay or determine the presence or concentration of a specific substance or analyte within a liquid sample suspected of containing that particular analyte. Such analysers are well known and almost universally employ some sort of a calibration curve that relates analyte concentration within a sample having a known analyte concentration against the signal generated by the reaction monitoring means in response to the presence of the analyte. Such samples are frequently called "calibrators" or "calibration solutions" or "standard solutions". For greatest accuracy, calibration curves are established at regular intervals, e.g. when a new batch of consumables is produced, to compensate for reagent particulars on individual analysers, and to compensate for equipment performance. The range of analyte concentrations used in establishing a full calibration curve is typically chosen to extend below and beyond the range of analyte concentrations expected to be found within biological samples like blood, blood serum, blood plasma, urine and the like.

It is regular practice within the biochemical analytical industry to establish a full calibration curve for a chemical analyser by using multiple calibration solutions or calibrators which have been carefully prepared with known, pre-determined concentrations of analyte. These calibration or standard solutions are assayed one or more times and the mean resulting reaction signals are plotted versus their respective known analyte concentrations. A continuous calibration curve is then produced using any of several mathematical techniques chosen to produce an accurate replication of the relationship between a reaction signal and the analyte concentration. The shape of the calibration curve is affected by a complex interaction between reagents, analyte and the analyser's electromechanical design. Thus, even if the theoretical analyte-reagent reaction is known, it is generally necessary to employ mathematical techniques to obtain an acceptable calibration curve.

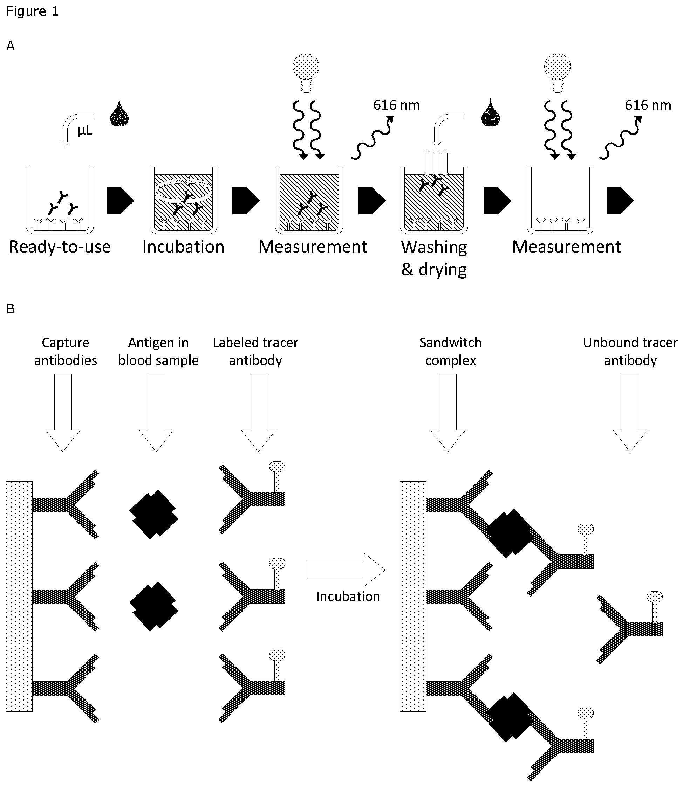

Most ways to determine the concentration of an analyte are immunoassay-based, i.e. the analyte is detected using immunoreagents such as antibodies, e.g. in a so-called "sandwich assay" (see e.g. Figure 1 B for an overview).

Nowadays, the immunoassays carried out in a laboratory have been subject to extensive automation and the demand for automated immunoassays is increasing. In general, there are two necessary components for such automation, namely the device, often also referred to as the "analyser", to determine the concentration in the sample and the (analyte-specific) assays that can be used with the respective device. Typically, the respective assays can be performed using commercially available ready-to-use kits that contain all the necessary reagents that can be applied in the respective device. Ideally, the device "as a platform" is suitable to be used together with various different ready-to use immunoassay kits in an automated way in order to ensure a wide applicability of the system.

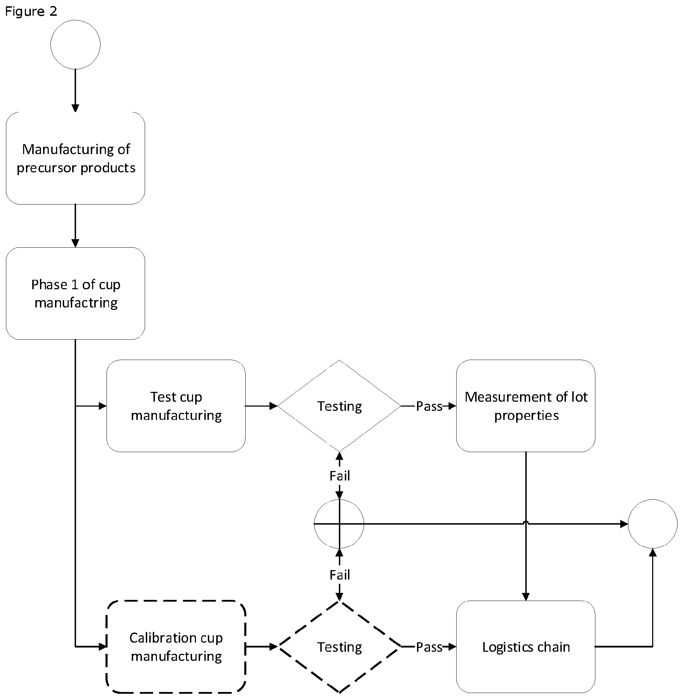

The commercially available reagents, e.g. containing the composition containing the immunoassay reagents, have typically been tested and calibrated by the manufacturer in-house to ensure that the assay provides the desired result for a sample containing a known concentration of the analyte. This calibration reduces the "batch-to-batch" variability of the immunoassays. This is also shown in Figure 2.

However, the in-house calibrations of the assay kits are typically all performed at the manufacturer's site and thus cannot account for "device-to-device" variations, i.e. variations between two individual devices of the same build, that might be due to minor variations e.g. in the detection sensitivity of the device. Thus, it is necessary to also perform a device-specific calibration.

Therefore, in order to avoid "device-to-device" variability of the immunoassays, the specific device is adjusted to the specific device by providing a further calibration adjustment kit, typically containing blank samples, for determining the background, as well as a sample containing a known concentration of the analyte and the device need to provide the desired result for said analyte using the specific device.

This adjustment has to be done regularly. This way it is ensured that the adjustment inter alia avoids or accounts for any drift effects of the detection sensor in the device. Further, the device-specific variations may also be slightly different for the different analyte assays or may also be affected by "batch-to-batch" variability of the immunoassays so that the device-specific adjustments should ideally be repeated each time a new batch of immunoassay is used in order to obtain the most accurate results.

One example of a device is the AQT90 FLEX immunoassay analyser (Radiometer Medical ApS), which is a platform device that can be used in combination with different ready-to-use immunoassay kits for specific biomarker testing in blood or blood plasma samples. Each batch of the ready-to-use immunoassay kits is calibrated by the manufacturer and the user has to perform a device-specific calibration of the individual AQT90 FLEX immunoassay analyser using batch-specific calibration cartridges that inter alia contain samples having a known concentration of an analyte.

EP 0 892 927 B1 discloses a one-step all-in-one dry reagent immunoassay and a device for using a respective immunoassay.

U.S. 3,960,497 discloses the basic concepts of calibrating and verifying the calibration of a chemical analyser using standard solutions having known values of the particular characteristics being measured.

US 6,277,584 discloses a method for calibrating a chemical analyser with improved accuracy at low signal levels.

Kunz et al. "Addressing the challenges of biomarker calibration standards in ligand-binding assays: a European Bioanalysis Forum perspective", Bioanalysis (2017) 9(19), 1493-1508 summarizes various ways of calibrating biomarker assays and calibration standards.

WO 2014/143323 A1 discloses a method for internal calibration of an assay that measures the concentration of an analyte use an immunoassay.

However, the need to provide an individual device calibration is accompanied by several disadvantages. Since the adjustment kit needs to contain a sample having a known concentration of an analyte, the production of the kits is rather expensive and often have a limited stability, e.g. due to a limited stability of the analyte. Moreover, the adjustment kit usually also contains separate blank, i.e. background, samples that do not contain the analyte, which of course increase the number of samples needed for the adjustments as well as the time to perform the adjustment measurements. Further, the customer has to obtain the respective adjustment kits. In other words, if the customer has not ordered the respective adjustment kit for the device, it is possible that, depending on the setup of the device, he either cannot perform the automated assay at all or he may obtain inaccurate results due to missing on-site device adjustment.

There is thus a need for alternative ways to provide the adjustment of specific devices, which does not require the use of a separate adjustment kit comprising a sample having a known concentration of an analyte.

It is thus an object of the present invention to provide methods for specific individual device adjustment that do not require the use of an analyte-containing sample having a known concentration of the analyte.

It is consequently also an object of the present invention to provide methods for determining the concentration of an analyte using a respective calibrated device.

## SUMMARY OF INVENTION

The object is solved by a method of calibrating an immunoassay device, the device being suitable for determining the concentration of an analyte in a sample by contacting the sample with a measuring cell selected from a batch of identical measuring cells, wherein each of the identical measuring cells contains a capture antibody immobilized on the inner surface of the respective measuring cell and a tracer antibody, and measuring a sample signal from the measuring cell using the device,

wherein the method comprises the steps of

     a) providing one or more measuring cells from the batch of identical measuring cells;

     b) adding assay buffer to the one or more measuring cells;

     c) measuring a first signal for each of the one or more measuring cells;

     d) washing the one or more measuring cells;

     e) optionally drying the one or more measuring cells;

     f) measuring a second signal for each of the one or more measuring cells; and

     g) calculating one or more device-specific correction factors for determining the concentration of an analyte in a sample using the one or more measurements of the first signal and the one or more measurements of the second signal as defined by claim 1.

The object is further solved by a method of determining the concentration of an analyte in a sample, the method comprising the steps of

     i) calibrating an immunoassay device according to the calibration method described herein using one or more measuring cells from a batch of identical measuring cells, wherein each of the identical measuring cells contains a capture antibody immobilized on the inner surface of the respective measuring cell and a tracer antibody, the calibration method thereby providing one or more device-specific correction factors;

ii) providing a sample containing the analyte;

iii) contacting the sample with a measuring cell selected from the batch of identical measuring cells;

iv) washing the measuring cell;

v) optionally drying the measuring cell;

vi) measuring a sample signal from the measuring cell using the immunoassay device; and

vii) calculating the concentration of the analyte from the sample signal and at least the one or more device-specific correction factors, as defined by claim 10.

## DETAILED DESCRIPTION

As used herein, the term "a", such as in "a sample" may refer to the singular or to the plural. The meaning is thus the same as "one or more".

The term "comprising" as used herein also encompasses, as a preferred embodiment, the term "essentially consisting of" or even as "consisting of".

As used herein, the term "about" in connection with a number, such as in "about 70°C" encompasses the exact number as well as minor differences that are however e.g. due to inaccuracies of the measuring method for e.g. the temperature. Typically, the term "about" encompasses a value that is also 10 % higher or 10 % lower than the exact number. For example, the term "about 70 °C" then also includes temperatures from 63 to 77°C. In a preferred embodiment, the term "about" encompasses a value that is 5 % higher or 5 % lower than the exact number. In another preferred embodiment, the term "about" encompasses a value that is 1 % higher or 1 % lower than the exact number.

As used herein, the term "capture antibody" means an antibody that is immobilized, i.e. on the inner surface of a measuring cell that thus "captures" an analyte that is thereby also immobilized/bound to the inner surface of the measuring cell. Thus, the capture antibody is directed against the analyte of interest as the antigen. The capture antibody may be immobilized to the surface of the measuring cell covalently or non-covalently. In one embodiment, the immobilization relies on the interaction between biotin and streptavidin. For example, the capture antibody may be biotinylated and the inner surface of the measuring cell may be coated with covalently bound streptavidin. The capture antibody is then immobilized on the inner surface of the measuring cell through the interaction between biotin and streptavidin. The interaction between biotin and streptavidin is so strong that it is essentially irreversible. However, other immobilization methods are equally suitable to immobilize the capture antibody. The capture antibody is however not removed or substantially removed from the inner surface of the measuring cell during the washing, incubation and drying steps of an immunoassay.

As used herein, the term "tracer antibody" refers to an antibody that is labelled with a detection label. The tracer antibody is directed against the analyte or complex of analyte and capture antibody. The label may then subsequently e.g. provide a detectable signal using an appropriate sensor, e.g. a colorimetric signal or a fluorescence signal. The label may be attached covalently or non-covalently to the tracer antibody. Examples for labels attached to the antibody include, but are not limited to, fluorescent tags such as green fluorescent protein or variants thereof, enzymatic tags such as galactosidase that catalyses a colorimetric reaction, radioactive isotopes, small molecule fluorescent or phosphorescent dyes, other small molecule-coloured dyes, nanoparticles and the like. Examples of the label in particular include, but are not limited to, lanthanide chelate labels e.g. as described in in EP 0 892 927 B1 or US 9,944,657. The tracer antibody binds to the analyte forming a ternary complex between the analyte, tracer antibody and capture antibody (see Figure 1).

It will be appreciated that the term "antibody" when used herein in the context of the terms "capture antibody" and "tracer antibody" is used in the conventional sense within the field of immunoassays, i.e., for example to encompass monoclonal antibodies, polyclonal antibodies as well those fragments of antibodies that binds to a relevant epitope of the analyte (e.g. antigen), etc. Further, antibodies (or fragments thereof) may be produced in e.g. animals or in cell lines, including utilizing recombinant techniques and synthetic or semi-synthetic methodologies.

As used herein, the term "calibration of a device", "calibration of an immunoassay device", "calibrating an immunoassay device" or "calibrating a device" refers to deriving the correction factors required for the fine-tuning of an individual immunoassay device (hereinafter "a device") for determining the concentration of an analyte in a sample with high accuracy.

As used herein, the term "measuring cell" refers to a container that contains the components to determine the concentration of an analyte in a sample, i.e. a container comprising the tracer antibody and the capture antibody and optionally stabilizing agents and further assay components. Further, the measuring cell is configured in a way that the signal generated by the label of the tracer antibody can be detected. The measuring cell may be non-disposable or disposable. In an automated system, the measuring cells are typically disposable and configured for use in connection with a respective device for determining the concentration of the analyte. Typically, the measuring cells are for one-time use only. It is preferred that the measuring cell is a ready-to-use measuring cell, i.e. a measuring cell that need not be manually modified by the user for the measurement.

As used herein, the term "bodily fluid" means any kind of fluid that is generated by a higher organism. Non-limiting examples include blood, blood plasma, blood serum, urine, saliva and cerebrospinal fluid. In a preferred embodiment, the bodily fluid originates from a mammal, preferably a human.

As used herein, the term "correction factor" refers to a calculation constant that is used to calculate the concentration of an analyte from a measured signal, e.g. a fluorescence signal. The correction factor may be an additive constant or a multiplicative constant. The factor may be a positive number or a negative number. Correction factors may be device-specific or measuring cell-specific, or both. For example, device-specific correction factors may depend on the sensitivity of the instrument, which can slightly vary even between two embodiments of the same model of devices as a "device-to-device" variation. Measuring cell-specific correction factors may inter alia depend on the nature of the components of the measuring cell, including the tracer antibody and the capture antibody. A measuring cell-specific correction factor may thus depend for example on the affinity of the antibody for the analyte, presence or absence of stabilizing agents, the nature of the label of the tracer antibody, the amount of capture antibody and tracer antibody in the measuring cell and/or the material of the inner surface of the measuring cell. Measuring cell-specific correction factors are typically independent of the specific individual device that is used for the measurement. Measuring cell-specific correction factors are thus typically determined by measuring a sample having a known concentration of an analyte on multiple devices and the results may be averaged to determine the measuring cell-specific correction factors.

As used herein, the term "substantially" as in e.g. "substantially remove", "substantially dry" or "substantially differ" means that it allows minor deviations from the absolute that are however negligible. In a preferred embodiment, the term "substantially" also includes at most a 10 % deviation. As an illustrative example, this would mean that "substantially remove" means that at least 90 % will be removed. "Substantially dry" in this illustrative example mean that the sample is at least 90 % dry so less than 10 % of the inner surface of the measuring cell is covered by a fluid. In another preferred embodiment, the term substantially includes at most a 5 % deviation.

As used herein, the term "dry" means that the measuring cell does not contain a fluid, i.e. the inner surface of the measuring cell is not covered by a fluid.

As used herein, the terms "device" and "analyser" are used interchangeably.

As used herein, an "automated device" is a device for determining the concentration of an analyte that functions in an automated fashion. For example, such a device may contain a sampler that automatically applies the required amount of sample to the measuring cell, automatically performs the incubation steps, washing steps signal detection steps and/or automatically calculates the concentration based on the obtained measurements.

As used herein, the term "individual device" refers to the specific device to be calibrated, i.e. the exactly one device that is calibrated, namely a device with a unique identifier. Other devices for determining the concentration of an analyte in a sample may have the same build and same setup but are still not the "same individual device" and instead devices of the same series of devices or devices of the same device type. For example, an individual device would be a specific AQT90 FLEX immunoassay analyser at the laboratory of the user. Other AQT90 FLEX immunoassay analysers, e.g. those that are used at the manufacturer's site not at the user's laboratory, are devices from the same "series of devices" but they are not the same "individual device" of the user.

As used herein, the term "series of devices" means that there are a multitude of different devices of the same build and setup, e.g. the AQT90 FLEX immunoassay analyser, but only one AQT90 FLEX immunoassay analyser is an "individual device".

*The method of calibration*

The present invention relates to a method of calibrating an immunoassay device, the device being suitable for determining the concentration of an analyte in a sample by contacting the sample with a measuring cell selected from a batch of identical measuring cells, wherein each of the identical measuring cells contains a capture antibody immobilized on the inner surface of the respective measuring cell and a tracer antibody, and measuring a sample signal from the measuring cell using the device,
wherein the method comprises the steps of

a) providing one or more measuring cells from the batch of identical measuring cells;

b) adding assay buffer to the one or more measuring cells;

c) measuring a first signal for each of the one or more measuring cells;

d) washing the one or more measuring cells;

e) optionally drying the one or more measuring cells;

f) measuring a second signal for each of the one or more measuring cells; and

g) calculating one or more device-specific correction factors for determining the concentration of an analyte in a sample using the one or more measurements of the first signal and the one or more measurements of the second signal.

An overview of an embodiment of the inventive method is also illustrated in the scheme of Figure 1 A wherein step a) is illustrated as the first measuring cell ("cup") from the left ("Ready-to-use"); step b) is illustrated as the drop ("mL") added to the first measuring cell; the "Incubation" is the time after the addition of the assay buffer allowing complete wetting and dissolution of components (e.g. the tracer antibody); step c) is illustrated as the third measuring cell from the left ("Measurement"); steps d) and e) are illustrated as the fourth measuring cell from the left ("Washing & drying"), and step f) is illustrated as the measuring cell to the far right ("Measurement"). Step g) is conducted subsequently based on the first signal(s) and the second signal(s).

The method of calibrating the device is typically to be performed before the device is used for determining the concentration of an analyte in a sample. It is preferred that the device is an automated device. It is also preferred that the device is suitable for use in combination with ready-to-use immunoassay kits for determining the concentration of an analyte and even more preferably in combination with ready-to-use measuring cells containing reagents of said immunoassay.

Further, it is preferred that other devices exist that are of the same build as the device of the method of the present invention. Thus, in a preferred embodiment, the device to be calibrated is an individual device. The individual device is of a series of devices of the same build that may only differ in small details, such as the sensitivity of detection sensor, e.g. in the optical unit such as a photomultiplier tube. One or more of said other devices may have been used for additional calibration experiments that optionally may have used a sample containing a known concentration of an analyte.

The steps of the method of the invention are followed in the order as described above, but the method may optionally contain further method steps before, after and/or between method steps a) to g).

The one or more measuring cells in **step a)** are analyte-free. Thus, the method of the present invention provides a calibration of a device without having to use an analyte-containing sample having a known (i.e. non-zero) concentration of the analyte. The calibration of the device can thus be performed with measuring cells that are the same as the measuring cells to be used for determining the concentration of an analyte in a sample, i.e. the one or more measuring cells used for the calibration are selected from the same batch of identical measuring cells as the measuring cell used for determining the concentration of an analyte in a sample.

In a preferred embodiment, the measuring cell has the shape of a cup. Each cup has an open end and an inner surface. At least part of the inner surface is coated with a capture antibody. The cups, as well as a device for holding a plurality of the cups are e.g. described in US 7,922,986 B2.

The cup may be individually closed or sealed before use, there being no fluid communication between any of the cups.

In a preferred embodiment, the one or more measuring cells in step a) are substantially dry or dry, i.e. does not contain a fluid such as an assay buffer. This means that all reagents, e.g. the capture antibody and the tracer antibody are present in a dry state and e.g. the tracer antibody or other components need to be reconstituted in order to be in solution in the measuring cell.

Since the measuring cell contains a capture antibody and a tracer antibody, the measuring cell contains components suitable for determining the concentration of an analyte in a sample, apart from the actual sample.

In a preferred embodiment, the measuring cell is a ready-to-use measuring cell.

In another preferred embodiment, the measuring cell, e.g. a ready-to-use measuring cell that may e.g. be from a specific measuring cell batch containing all dry components of the respective bioassay, had previously been calibrated, e.g. by using at least one ready-to-use measuring cell from the same batch, using at least one device from the same series of devices as the individual specific device to be calibrated according to the present inventive method. The calibration of the measuring cell may occur by using one or more samples containing an analyte in a known concentration. In one embodiment, the measuring cell had been calibrated using a master calibration curve, wherein each sample contributing to the master calibration curve has a different but known concentration of an analyte.

In another preferred embodiment, the measuring cells are produced in batches. It is further preferred that the production procedure of said batches ensures that all measuring cells of a batch will give equal or substantially equal response when measured with the same sample, i.e. that the batch of measuring cells for all practical purposes are "identical". When the next batch is produced, the response of these measuring cells will also give equal responses with the sample, but the response may vary from the response of the previous batch, also called "batch-to-batch variation". It is further preferred that each batch of measuring cells is calibrated respectively.

In the method of calibrating according to the present invention, the measuring cells used for calibration of the device and the measuring cell used for (subsequently) determining the concentration of an analyte in a sample using the calibrated device are selected from a batch of identical measuring cells, wherein each of the identical measuring cells contains a capture antibody immobilized on the inner surface of the respective measuring cell and a tracer antibody.

As explained above, the term "identical" is intended to mean that the individual measuring cells are produced under the same conditions and are intended to have a substantially identical composition and to exhibit a substantially identical performance. In some embodiments, such a batch of identical measuring cells are from one or more production batches, preferably from the same production batch.

Based on the measurement of the measuring cell calibration, a measuring cell specific correction factor can be calculated, and this measuring-cell correction factor can account for batch-to-batch specific adjustments that can be communicated to an individual device e.g. via a bar-code on the measuring cell or the cartridge containing multiple measuring cells (also called a factory-defined calibration data barcode). In such an embodiment, the device to be calibrated then comprises a respective barcode reader. In an alternative embodiment, the device to be calibrated may provide means to manually input the measuring cell specific correction factor.

In a preferred embodiment, more than one measuring cell is used for the calibration method according to the present invention. Without being bound to theory, the more measuring cells are used, the more accurate the final calculation of the one or more device-specific correction factors will be. However, the number of measuring cells to achieve sufficient accuracy may also depend on the particulars of the measuring cell, e.g. the capture antibody, the tracer antibody, the material of the measuring cell and the like.

In another preferred embodiment, when more than one measuring cell is used for the calibration, measuring cells, herein also called outliers, where the subsequent first and second signal substantially differ from the first and second signals obtained for the other measuring cells, the measurements of said outliers are not included in the calculation of step g).

In a preferred embodiment, the method is performed using from about 4 to about 16 measuring cells.

In another preferred embodiment, the measuring cell has a reaction volume from 10 to 200 $\mu$L and preferably from about 20 to about 100 $\mu$L.

The assay buffer in **step b)** can be any assay buffer. In a preferred embodiment, the assay buffer is an aqueous buffer. A suitable buffer preferably contains buffering agents, salts and detergents. In a preferred embodiment, the buffer has a pH from about 6.5 to about 8 and preferably from about 7.0 to about 7.8. It is particularly preferred that the buffer has a pH of about 7.4. For example, in one embodiment, the assay buffer is the commercial buffer of the AQT90 FLEX Solution Pack (Radiometer).

In a preferred embodiment, the assay buffer comprises buffer agent, salt and detergent.

In step b), the volume of added assay buffer is usually the same or substantially the same for each of the one or more measuring cells within an assay. When adding the assay buffer to the preferably dry measuring cell, the capture antibody preferably remains immobilized on the surface, but the tracer antibody is dissolved within the assay buffer.

In a preferred embodiment, step b) also contains, after the addition of the assay buffer, an incubation step. Without being bound to theory, the incubation step ensures that the tracer antibody dissolves in the assay buffer.

In one embodiment, the incubation time is from about 1 to about 15 min, preferably from about 2 to about 10 min. The incubation temperature can be any temperature and is preferably from about 4 °C to about 40°C, more preferably from about 22 °C to about 38°C. In a particularly preferred embodiment, the temperature is about 37°C.

In another preferred embodiment, the sample is being mixed during incubation. In a particularly preferred embodiment, the sample is mixed for the full duration of the incubation. The total amount of mixing should preferably be the same for all measuring cells of a measurement.

In **step c)** the first signal is measured for each of the one or more measuring cells by using a suitable detection sensor. While measuring, the tracer antibody is in solution in the assay buffer and generates the detectable signal. In a preferred embodiment, the determined signal is a colorimetric signal, a phosphorescent signal or a fluorescent signal. In a preferred embodiment, the first signal is a fluorescent signal.

It is particularly preferred that the first signal is a time-resolved fluorescence signal. Such a signalling system of time-resolved fluorescence is for example described by Hemmilä, In Johnstone AP, Turner MW, eds. Immunochemistry 1: a practical approach. Oxford: IRL Press, 1997, pages 193-214. Suitable labels for the tracer antibody for time-resolved fluorescence include lanthanide chelate labels that are covalently attached to the antibody part of the tracer antibody. Examples of suitable lanthanide chelate labels are e.g. described in EP 0 892 927 B1 or US 9,944,657. In a preferred embodiment, the label is a europium-based lanthanide chelate.

Since none of the tracer antibody in the measuring cell is removed before measuring the first signal in step c), the first signal generated in step c) can, without being bound to theory, represent a good approximation of the maximum signal level for the device without having to use a specific analyte containing calibrator for the measurement.

In **step d),** the one or more measuring cells are washed. In a preferred embodiment, the assay buffer is used as wash buffer for the washing. The washing step d) thus removes the tracer antibody from the measuring cell. The washing step may be repeated, if necessary, to remove or at least substantially remove all of the tracer antibody from the measuring cell.

The washing step usually includes aa) removing the fluid from the measuring cell, e.g. by using negative pressure and bb) adding the buffer that is used for the washing to the measuring cell and cc) again removing the fluid from the measuring cell.

In one embodiment, step d) comprises one or more washing steps, where steps aa), bb) and (optionally cc)) are repeated.

In a preferred embodiment, the washing in step d) is performed in a way that at the end of the one or more washing steps, the fluid is removed or substantially removed from the measuring cell e.g. using negative pressure, i.e. most of the fluid is already removed before step e).

In **step** e), the measuring cell is optionally dried after the washing to further remove remaining remnant fluid. The drying may occur by any known drying method. Without being bound to theory, the measuring cells typically contain some residual fluid after the washing step, even if the wash buffer had been substantially removed in substep cc) of step d). In some cases, it may be found that the measuring cell is sufficiently "dry" after step d), and that the amount of residual fluid (like a thin fluid film on the inside of the measuring cell) will not have any significant impact on the measurement of the second signal (step f)). In some other case it may be found that a separate drying step is preferred.

In a preferred embodiment, the measuring cells are air-dried. It is particularly preferred that the air that is used for the air-drying is filtered before containing the measuring cells. Without being bound to theory, the filtering of the air removes contaminants that may influence the accuracy of the measurements.

In another preferred embodiment, the air for the air-drying has a temperature of about 50 °C to about 90 °C. It is particularly preferred that the air has a temperature of about 70 °C.

In another preferred embodiment the air-drying occurs by blowing the air into and through the measuring cell at a speed of 5 L/min.

After the drying, the measuring cell is dry, i.e. the residual volume after emptying the measuring cell is preferably less than 6 µL fluid and even more preferably has been dried completely.

Without being bound to theory, the measuring cell then still contains the immobilized capture antibody after drying, that has not been removed during the washing.

In **step** f), a second signal for each of the one or more measuring cells is determined on the dry measuring cell obtained in step e) using the same detection sensor as in step c), thereby the second signal is the same type of signal as the first signal measured in step c).

In a particularly preferred embodiment, the first signal and the second signal are fluorescence signals, preferably time resolved fluorescence signals.

The first signal and the second signals may be detected using any kind of detection sensor. Non-limiting examples of detection sensors are optical detection units or radioactivity detection units. Non-limiting examples of optical detection units are avalanche phot diodes, photomultiplier tubes and the like. It will be understood that preferably the detection sensor is the exact same for measuring the two signals.

In yet another particularly preferred embodiment, the first signal and/or the second signal are detected by a photomultiplier tube (PMT) of the device. It is commonly known that the sensitivity and background signals of photomultiplier type may vary between different PMT of the same build, and this may be one of the reasons for inter-device variations.

However, in contrast to the first signal generated in step c), the second signal is generated using the dry measuring cell obtained from step e). Since the tracer antibody had been removed from the measuring cell in step d) and optionally in step e), the second signal, without being bound to theory, should be significantly lower than the first signal and could thus be considered as a device-specific blank or background signal.

It was surprisingly found that this signal could be used even though it was obtained from the dry state of the measuring cell while the first signal was obtained from a "wet" state of the measuring cell containing assay buffer as medium.

Having obtained a first high signal and a second low signal in a measuring cell, without having to provide the analyte in a known concentration in the measuring cell, it is possible in **step g)** to calculate one or more individual device-specific correction factors for determining the concentration of an analyte in a sample using the one or more measurements of the first signal and the one or more measurements of the second signal.

As described above for the measuring-cell correction factors, the device-specific correction factors can be used in an algorithm to more precisely convert the measured signal for an analyte into the respective analyte concentration. For example, when the measured signal is a fluorescence signal, the obtained fluorescence photon count, e.g. in a photomultiplier tube as detection sensor, needs to be converted into an analyte concentration.

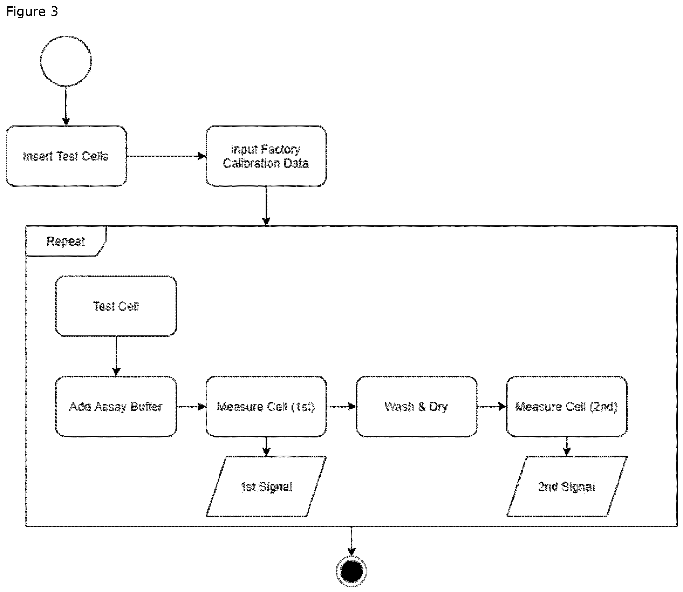

In a preferred embodiment, in this algorithm, also the measuring cell-specific correction factors may be taken into account. This is in one example also illustrated in Figure 3.

In a more preferred embodiment, the second signal obtained in step f) is used as an additive constant device-specific correction factor. In another preferred embodiment, the first signal obtained in step c) is used as a multiplicative constant device-specific correction factor.

In a particularly preferred embodiment, the following calculation can be applied to determine the concentration of a sample:

$$\text{Equation 1:} \qquad M = a*R(c) + b$$

In Equation 1, "M" is the measured signal. The factor "a" is the device-specific sensitivity correction factor for the device derived from the measurement in step c), i.e. from the first signal. "R(c)" is the response function over concentration which is preferably a strictly increasing monotonic C2 function. The response function is however a function that is independent from the specific device. The additive "b" is the "background" signal for the device and is derivable from the second signal obtained in step f).

In another preferred embodiment, the calculation of the device-specific correction factors comprises a correlation of the measured first and second signal of the individual device to the respective first and second signal obtained for one or more different devices from the same series, herein also called "master device(s)", that have been used to determine the measuring cell specific correction factors. Said device-specific correction factors of the master device may thus also be the average correction factors of several master devices. In a preferred embodiment, the device-specific correction factors of the master device can be read by the device to be calibrated, e.g. via a barcode reader. In an alternative embodiment, the device-specific correction factors of the master device may be input to the individual device to be calibration manually by the user.

In a preferred embodiment, the calculation of the individual device-specific correction factors in step g) includes averaging the measured results for the first signal and second signal, respectively, when more than one measuring cell is used for the calibration in steps a) to f). In other words, when for example eight measuring cells are used for the calibration, eight first signals are averaged and eight second signals are averaged.

In a preferred embodiment, the calibration according to the present invention is performed for a device for determining the concentration of an analyte in a sample, wherein the sample is a bodily fluid.

In a preferred embodiment, the bodily fluid is whole blood, urine, blood plasma or blood serum.

In a particularly preferred embodiment, the bodily fluid is whole blood or blood plasma.

The analyte in the sample to be determined after the calibration may be any analyte of interest. In a preferred embodiment, the analyte is NT-proBNP (N-terminal prohormone of brain natriuretic peptide), Procalcitonin (PCT), BNP (brain natriuretic peptide), D-Dimer, cardiac Troponin I (cTnI), cardiac Troponin T (cTnT), beta-hCG (human Chorionic Gonadotropin), C-reactive protein (CRP), Myoglobin, or CK-MB (Creatinine kinase-muscle/brain).

*The method of determining the concentration of an analyte*

The present invention further relates to a method of determining the concentration of an analyte in a sample, the method comprising the steps of

i) calibrating an immunoassay device according to the calibration method described herein using one or more measuring cells from a batch of identical measuring cells, wherein each of the identical measuring cells contains a capture antibody immobilized on the inner surface of the respective measuring cell and a tracer antibody, the calibration method thereby providing one or more device-specific correction factors;

ii) providing a sample containing the analyte;

iii) contacting the sample with a measuring cell selected from the batch of identical measuring cells;

iv) washing the measuring cell;

v) optionally drying the measuring cell;

vi) measuring a sample signal from the measuring cell using the immunoassay device; and

vii) calculating the concentration of the analyte from the sample signal and at least the one or more device-specific correction factors.

The steps of the method of the invention are followed in the order as described above, but the method may optionally contain further method steps before, after and/or between method steps i) to vii). This being said, the order of steps i) and ii) is not particularly critical, and the sample, e.g. a blood sample, may in some cases be collected at a time prior to, or during, the calibration of the device.

The device, sample, analyte, capture antibody and tracer antibody are as described above for the calibration method of the present invention.

In one embodiment, **step ii)** may also contains a step of diluting the sample with assay buffer. The dilution will be accounted for in step vii) by introducing a dilution factor. Such a dilution step may be necessary depending on the concentration of the analyte in the sample and the specifics of the analyte assay. In other embodiments, the sample is used as is, for example a whole blood sample without dilution.

The measuring cell in **step iii)** is from the same batch of identical measuring cells as the one or more measuring cells used for the calibration in step i).

In step iii), the sample is contacted with (e.g. dispensed to, or added to) the measuring cell. Typically, an assay buffer is also added to the measuring cell either before or after (preferably before) dispensing or adding the sample.

In a preferred embodiment, step iii) also contains an incubation step. Without being bound to theory, the incubation step ensures that a complex between capture antibody, analyte and tracer antibody can form. In a particularly preferred embodiment, the incubation time is from about 1 to about 15 min and more preferably from about 2 to about 10 min.

The washing **step iv)** removes unbound tracer antibody from the sample. In a preferred embodiment, the wash buffer is the assay buffer described above. In another preferred embodiment, the washing step is the same as described herein-above for step d).

The optional drying **step v)** is in a preferred embodiment the same as described herein-above for drying step e) and may be preferred for the same reasons as explained herein-above for drying step e).

In **step vi)**, the sample signal that is measured from the measuring cell using the immunoassay device is the same type of signal as the first signal and/or second signal described above in step c) and f). In a preferred embodiment, the signal is thus a fluorescence signal, preferably a time resolved fluorescence signal.

In **step vii),** the sample signal is converted into an analyte concentration. As described above for step g), the calculation comprises a correction based on the device-specific correction factors determined in step i). In a preferred embodiment, the calculation also takes into account the measuring cell-specific correction factors, preferably the batch-specific measuring cell-specific correction factors as already explained above. In thus another preferred embodiment, Equation 1 is used for the calculation for the concentration of the analyte. In an embodiment the sensitivity, 'a' and the background 'b' from equation 1 are also used to apply one or more corrections depending on 'a' and 'b'.

In another preferred embodiment, the device contains a CPU to perform the above calculations. It is further preferred that the device is configured to display the calculated analyte concentration, e.g. on a display screen or as a print-out. Moreover, as already described above, it is preferred that the device comprises a barcode reader configured to receive bar-coded information such as one or more measuring-cell specific correction factor and/or master device-specific correction factor.

## BRIEF DESCRIPTION OF FIGURES

**Figure 1:** Figure 1 A describes an example of the "wet cup calibration", i.e. a method of device-specific calibration of the present invention. A ready-to-use measuring cell containing a capture antibody and a tracer antibody is provided and buffer is added. After an incubation period, fluorescence is measured at a desired wavelength, e.g. at 616 nm, depending on the fluorescence detection system. Next, the measuring cell is washed and dried and fluorescence is again measured for the dry measuring cell. Figure 1 B describes the principle of an immunoassay in a measuring cell to determine the concentration of an analyte, e.g. an antigen, in a sample. The immobilized antibody is the capture antibody. To this antibody, an analyte may bind. A ternary complex may then form, e.g. after an incubation, together with a tracer antibody, which is labelled with a detectable label that can be detected using an appropriate detection system, such as fluorescence and in particular time resolved fluorescence. Tracer antibody that is not bound to the analyte-capture antibody complex will however be removed by washing so that the tracer antibody signal indicates the amount of analyte in a sample.

**Figure 2** provides an overview of the calibration steps necessary for ready-to-use measuring cells containing bioassay reagents for an individual device. After the manufacturing of the measuring cells, the respective test cup (i.e. measuring cell) batch is tested for quality and it either passes or fails this test. If it passes the test, the batch-specific properties are determined. The same applies to the manufacturing of the calibration cups for the device-specific calibration, i.e. cups configured to perform a calibration, e.g. cups additionally comprising an analyte in a known amount or concentration. Also these calibration cups need to be tested and the calibration cup batch either passes or fails this test. However, such a test is not necessary when using the device-specific calibration of the present invention since the calibration can be performed without using such calibration cups and instead requires the use of the measuring cups that would also be suitable for applying the sample to be measured, i.e. a measuring cell not containing an analyte in a known amount or concentration.

**Figure 3** also shows an embodiment of the individual device-specific calibration of the present invention. The measuring cells are inserted into the individual device and the factory calibration data for the specific batch of measuring cells is input. Next, assay buffer is added to a measuring cell and the signal is measured. The measuring cell is then washed and dried and the signal of the dry measuring cell is measured. The data of the two measurements is device-specific and together with the input factory calibration data for the respective measuring cell batch, i.e. measuring cell-specific calibration data, the concentration of an analyte can be determined with measuring cells of the respective batch on the individual device.

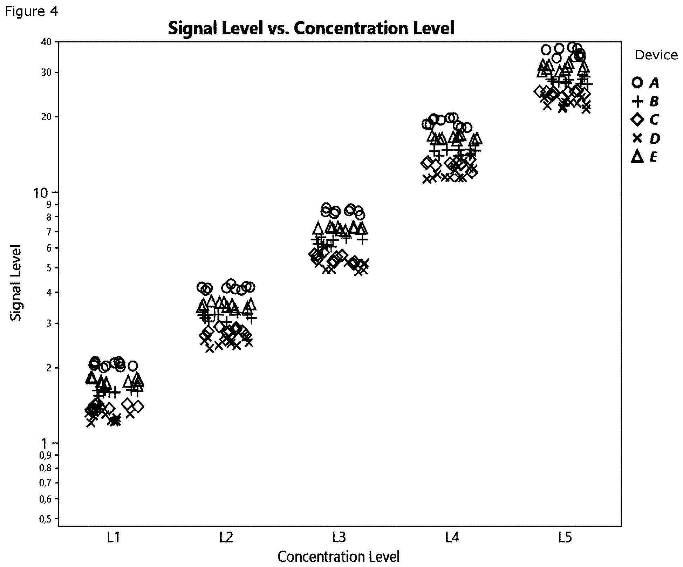

**Figure 4** shows the device reported signal level in arbitrary units for each of the samples L1 to L5 for each individual devices A to E in the absence of a device-specific calibration. All 10 replicates per device are shown. It can be seen that the signal level reported for the measured samples differ between the devices.

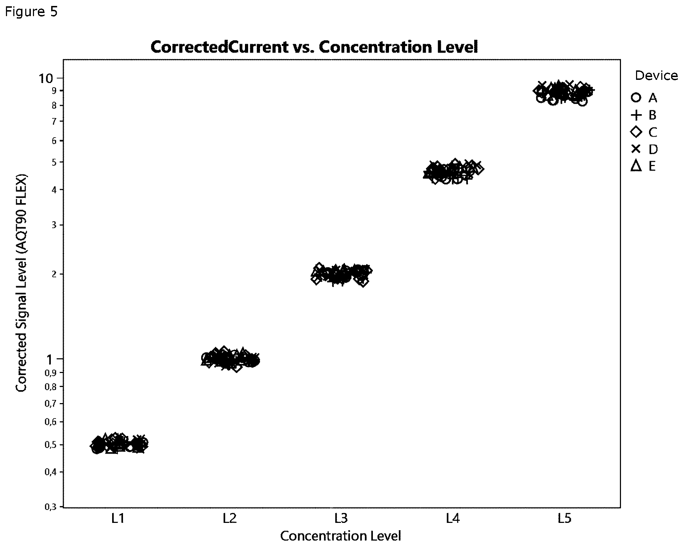

**Figure 5** shows the device reported signal level in arbitrary units for each of the samples L1 to L5 for each individual devices A to E after calibration using a calibration standard in a known concentration as is performed with the AQT90 FLEX immunoassay analyser. All 10 replicates per device are shown. When correcting the measured signal level using a standard calibration method (i.e. as for AQT90 FLEX) the differences between the individual devices are removed. Samples can thus be measured on different individual devices and the reported result will be the same.

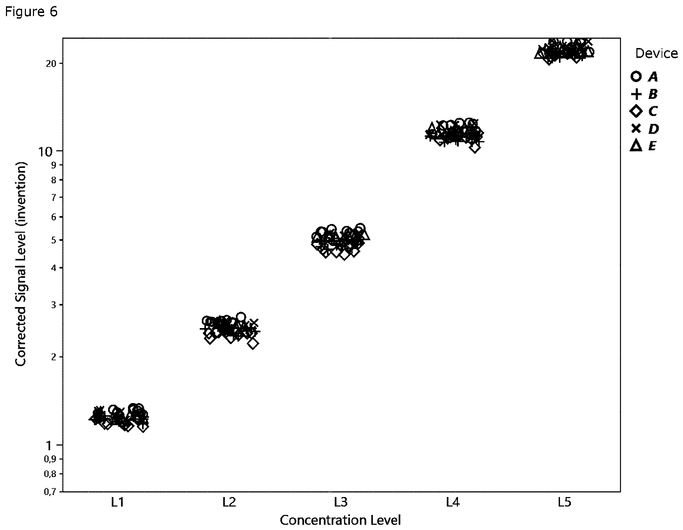

**Figure 6** shows the device reported signal level in arbitrary units for each of the samples L1 to L5 for each individual devices A to E after calibration using the "wet cup calibration" according to the present invention. All ten replicates per device are shown. When correcting the measured signal level using the wet cup calibration method of the present invention, the difference between the individual devices are removed. Samples can thus be measured on different individual devices and the reported result will be the same.

*Alternative embodiment*

In an *alternative* embodiment, which in the following will be disclosed with a reference to the main embodiments disclosed herein-above including the figures, one of both of the capture antibody and the tracer antibody is replaced by a "capture aptamer" or "tracer aptamer", respectively. Aptamers are single stranded DNA or RNA, or synthetic version hereof (XNA, like e.g. LNA) or hybrids thereof, e.g. selected from large random sequence pools. However, antibodies as well as aptamers can be chosen such as to be able to bind to a specific analyte (target molecule).

Hence, with respect to the *alternative* embodiment described immediately above, it shall be understood that within the present specification and claims any occurrence of the term "capture antibody" shall be understood as "capture aptamer", *mutatis mutandis,* and/or any occurrence of the term "tracer antibody" shall be understood as "tracer aptamer", *mutatis mutandis.* More generally, and when considering the combination of the main embodiments herein and the present *alternative* embodiment, it shall be understood that within the present specification and claims any occurrence of the term "capture antibody" shall be understood as either "capture antibody" or "capture aptamer" and any occurrence of the term "tracer antibody" shall be understood as either "tracer antibody" or "tracer aptamer", *mutatis mutandis.*

Moreover, the present invention, in the before-mentioned alternative embodiment, relates to a method of calibrating an immunoassay device, the device being suitable for determining the concentration of an analyte in a sample by contacting the sample with a measuring cell selected from a batch of identical measuring cells, wherein each of the identical measuring cells contains a capture antibody or a capture aptamer immobilized on the inner surface of the respective measuring cell and a tracer antibody or a tracer aptamer, and measuring a sample signal from the measuring cell using the device,

wherein the method comprises the steps of

a) providing one or more measuring cells from the batch of identical measuring cells;

b) adding assay buffer to the one or more measuring cells;

c) measuring a first signal for each of the one or more measuring cells;

d) washing the one or more measuring cells;

e) optionally drying the one or more measuring cells;

f) measuring a second signal for each of the one or more measuring cells; and

g) calculating one or more device-specific correction factors for determining the concentration of an analyte in a sample using the one or more measurements of the first signal and the one or more measurements of the second signal.

Additionally, the present invention, in the before-mentioned alternative embodiment, further relates to a method of determining the concentration of an analyte in a sample, the method comprising the steps of

i) calibrating an immunoassay device according to the calibration method described herein using one or more measuring cells from a batch of identical measuring cells, wherein each of the identical measuring cells contains a capture antibody or a capture aptamer immobilized on the inner surface of the respective measuring cell and a tracer antibody or a tracer aptamer, the calibration method thereby providing one or more device-specific correction factors;

ii) providing a sample containing the analyte;

iii) contacting the sample with a measuring cell selected from the batch of identical measuring cells;

iv) washing the measuring cell;

v) optionally drying the measuring cell;

vi) measuring a sample signal from the measuring cell using the immunoassay device; and

vii) calculating the concentration of the analyte from the sample signal and at least the one or more device-specific correction factors.

**EXAMPLES**

**Example 1 Comparison of measurements with and without device-specific calibration**

In the following, the calibration is adjusted to individual devices wherein the batch information and a reagent cartridge, containing the dry assay reagents in ready-to-use measuring cells, is used.

**Example 1.1 Calibration of a device**

A device configured for time resolved fluorescence was used for the calibration.
The general measurement protocol was as follows:
Measurements were performed in the presence ("wet measurement") or absence ("dry measurement") of a volume of 60 $\mu$L of suitable assay buffer having a pH of 7.4.
The measuring cells were incubated for 447 $\pm$ 10 s.
After the incubation, time resolved fluorescence was optionally measured ("wet measurement"), depending on the calibration method, using an LED current of 100 mA, light duration 400 $\mu$s, with an excitation wavelength of 340 nm, detection wavelength of 616 nm.
Next, the measuring cells were washed by repeated dispensing and emptying the measurement cell allowing time for removal of unspecific bound antigen as well as unbound material.
After the washing the measuring cells were dried by blowing 70 $\pm$ 5 °C with more than 3 L/min, preferably 5 L/min into the measuring cell for 30 s and after the drying the time resolved fluorescence was measured ("dry measurement").
The calibration was repeated with five individual devices (A, B, C, D and E) of the same series.

**Example 1.2 Measurement without device-specific calibration (comparative example)**

As reference samples, five lithium heparin plasma samples were prepared as standards by adding human myoglobin in five different concentrations (hereafter also called L1 to L5) of : 48.5 $\mu$g/L, L2: 97.4 $\mu$g/L, L3: 196 $\mu$g/L, L4: 471 $\mu$g/L, and L5: 1108 $\mu$g/L.
Said samples were applied to the device and a cartridge comprising the measuring cells for detecting human myoglobin was used. The measuring cells contained the same reagents as in the commercially available AQT90 FLEX Myoglobin test cups (AQT90 FLEX Myo test kit; Radiometer).

For each device, five myoglobin test cartridges were loaded and ten replicates per sample (L1, L2, L3, L4 and L5) were measured on each of the five devices.

No device-specific calibration was performed.

The results are summarized in Table 1 as the average of the ten replicates per each sample per device.

*Table 1*

| Concentration Level (arbitrary units) | A | B | C | D | E |
|---|---|---|---|---|---|
| L1 | 2.1 | 1.6 | 1.4 | 1.3 | 1.8 |
| L2 | 4.1 | 3.2 | 2.8 | 2.5 | 3.5 |
| L3 | 8.4 | 6.3 | 5.4 | 5 | 7.1 |
| L4 | 19 | 14.4 | 12.9 | 11.7 | 16.4 |
| L5 | 35.9 | 27.8 | 24.8 | 22.5 | 31.3 |

Further, the results are also shown in Figure 4.

The averages for L1 to L5 vary significantly between the different devices A to E.

**Example 1.3 The measurements are evaluated after calibration with the AQT90 FLEX immunoassay analyser calibration method using an analyte in a known concentration (comparative example)**

The devices A to E were calibrated with a device-specific calibration using the myoglobin calibration cartridge ("Myo CAL Cartridge", Radiometer, part no. 944-214) specific for the assay batch used for the measuring cells used in Example 1.2. The calibration cartridge contains two types of test measuring cells, namely measuring cells that, in addition to the dry assay reagents, also contain myoglobin in known concentration and respective measuring cells containing no myoglobin. The latter are used for determining the background signal.

The same assay buffer as above was used for the measurement of the device-specific calibration. Further, by scanning the barcode of the calibration cartridge, further pre-determined measuring-cell specific information for the calibration is communicated to the device.

After performing the above calibration measurements, the obtained calibration data are applied to the measurement data for L1 to L5 obtained in Example 1.2.

The results, i.e. the device-specific calibration corrected myoglobin levels of L1 to L5 are summarized in Table 2. The results are also shown in Figure 5.

*Table 2*

| Concentration Level (arbitrary units) | A | B | C | D | E |
|---|---|---|---|---|---|
| L1 | 0.5 | 0.5 | 0.5 | 0.5 | 0.5 |
| L2 | 1.0 | 1.0 | 1.0 | 1.0 | 1.0 |
| L3 | 2.0 | 2.0 | 2.0 | 2.0 | 2.0 |
| L4 | 4.6 | 4.5 | 4.7 | 4.6 | 4.7 |
| L5 | 8.6 | 8.7 | 9.0 | 8.9 | 8.9 |

When compared to the results in Table 1, there is less variation in the concentration levels between the devices A to E after device-specific calibration.

**Example 1.4 The measurements are evaluated after calibration with the wet cup calibration method (inventive)**

The devices A to E were calibrated with a wet cup calibration method using a calibration cartridge wherein the measuring cells contain no myoglobin, i.e. corresponding to the "background" or "blank" measuring cells used in the calibration cartridge used in Example 1.3.

For device-specific calibration, the background signal was determined by measuring the time-resolved fluorescence after the addition of the assay buffer ("wet measurement"), which was the same assay buffer as used in the examples above. Then, the measuring cells were dried and time-resolved fluorescence was determined of the dried measuring cells as a "dry measurement".

Both signals, i.e. the first signal of the measurement of the measuring cells containing the assay buffer and the second signal of the subsequent "dry measurement" were used for calculating the device-specific calibration factors "a" and "b". Said calibration factors were then applied to the experimental data of Example 1.2 using Equation 1.

The results are summarized in Table 3 and Figure 6 and indicate a low variability between the individual devices A to E, e.g. compared to the results without device-specific calibration.

*Table 3*

| Concentration Level (arbitrary units) | A | B | C | D | E |
|---|---|---|---|---|---|
| L1 | 1.3 | 1.2 | 1.2 | 1.3 | 1.3 |
| L2 | 2.6 | 2.5 | 2.4 | 2.5 | 2.5 |
| L3 | 5.3 | 4.9 | 4.7 | 5.0 | 5.1 |
| L4 | 12.0 | 11.1 | 11.1 | 11.7 | 11.7 |
| L5 | 22.7 | 21.4 | 21.3 | 22.5 | 22.3 |

**Claims**

1. A method of calibrating an immunoassay device, the device being suitable for determining the concentration of an analyte in a sample by contacting the sample with a measuring cell selected from a batch of identical measuring cells, wherein each of the identical measuring cells contains a capture antibody immobilized on the inner surface of the respective measuring cell and a tracer antibody, or wherein each of the identical measuring cells contains a capture aptamer immobilized on the inner surface of the respective measuring cell and a tracer aptamer, and measuring a sample signal from the measuring cell using the device,
   wherein the method comprises the steps of

   a) providing one or more measuring cells from the batch of identical measuring cells;
   b) adding assay buffer to the one or more measuring cells;
   c) measuring a first signal for each of the one or more measuring cells;
   d) washing the one or more measuring cells;
   e) optionally drying the one or more measuring cells;
   f) measuring a second signal for each of the one or more measuring cells; and
   g) calculating one or more device-specific correction factors for determining the concentration of an analyte in a sample using the one or more measurements of the first signal and the one or more measurements of the second signal.

2. The method of claim 1, wherein the one or more measuring cells are analyte free.

3. The method of any one of claims 1 to 2, wherein the method does not include the use of an analyte-containing sample having a known concentration of the analyte.

4. The method of any one of claims 1 to 3, wherein the sample is a bodily fluid, preferably the bodily fluid is whole blood, urine, blood plasma or blood serum, in particular the bodily fluid is whole blood or blood plasma.

5. The method of any one of claims 1 to 4, wherein the analyte is selected from the group consisting of NT-proBNP (N-terminal prohormone of brain natriuretic peptide), Procalcitonin (PCT), BNP (brain natriuretic peptide), D-Dimer, cardiac Troponin I (cTnI), cardiac Troponin T (cTnT), beta-hCG (human Chorionic Gonadotropin), C-reactive protein (CRP), Myoglobin, and CK-MB (Creatinine kinase-muscle/brain).

6. The method of any one of claims 1 to 5, wherein the first signal and the second signal are fluorescence signals.

7. The method of any one of claims 1 to 6, wherein the first signal and the second signal are detected by a photomultiplier tube of the device.

8. The method of any one of claims 1 to 7, wherein step b) comprises an incubation step after the addition of the assay buffer.

9. The method of claim 6, wherein the fluorescence signals are a time resolved fluorescence signal.

10. A method of determining the concentration of an analyte in a sample, the method comprising the steps of

i) calibrating an immunoassay device according to the method of any of claims 1 to 9 using one or more measuring cells from a batch of identical measuring cells, wherein each of the identical measuring cells contains a capture antibody immobilized on the inner surface of the respective measuring cell and a tracer antibody, or wherein each of the identical measuring cells contains a capture aptamer immobilized on the inner surface of the respective measuring cell and a tracer aptamer, the calibration method thereby providing one or more device-specific correction factors;
ii) providing a sample containing the analyte;
iii) contacting the sample with a measuring cell selected from the batch of identical measuring cells;
iv) washing the measuring cell;
v) optionally drying the measuring cell;
vi) measuring a sample signal from the measuring cell using the immunoassay device; and
vii) calculating the concentration of the analyte from the sample signal and at least the one or more device-specific correction factors.

11. The method of claim 10, wherein the calculation of the concentration of the analyte further includes one or more measuring cell-specific correction factors.

12. The method of any one of claims 1 to 11, wherein the following calculation can be applied to determine the concentration of a sample:

$$M = a*R(c) + b$$

wherein

- M is a measured signal,
- the factor $\alpha$ is a device-specific sensitivity correction factor for the device derived from the first signal from the measurement in step c),
- $R(c)$ is a response function over concentration that is independent from the specific device, and
- the additive b is the background signal for the device and is derivable from the second signal obtained in step f).

13. The method of any one of claims 1 to 11, wherein the calculation of the device-specific correction factors comprises a correlation of the measured first and second signal of the device to respective first and second signal obtained for one or more different devices from the same series that have been used to determine measuring cell specific correction factors.

**Patentansprüche**

1. Verfahren zum Kalibrieren einer Immunoassay-Vorrichtung, wobei die Vorrichtung geeignet ist, die Konzentration eines Analyten in einer Probe zu bestimmen, indem die Probe mit einer Messzelle in Kontakt gebracht wird, die aus einer Charge identischer Messzellen ausgewählt ist, wobei jede der identischen Messzellen einen auf der Innenfläche der jeweiligen Messzelle immobilisierten Fänger-Antikörper und einen Tracer-Antikörper enthält, oder wobei jede der identischen Messzellen ein auf der Innenfläche der jeweiligen Messzelle immobilisiertes Capture-Aptamer und ein Tracer-Aptamer enthält, und wobei ein Probensignal aus der Messzelle unter Verwendung der Vorrichtung gemessen wird,
wobei das Verfahren die Schritte

a) Bereitstellen einer oder mehrerer Messzellen aus der Charge identischer Messzellen;
b) Hinzufügen von Assaypuffer zu der einen oder den mehreren Messzellen;
c) Messen eines ersten Signals für jede der einen oder mehreren Messzellen;
d) Waschen der einen oder mehreren Messzellen;
e) optionales Trocknen der einen oder mehreren Messzellen;
f) Messen eines zweiten Signals für jede der einen oder mehreren Messzellen; und

g) Berechnen eines oder mehrerer vorrichtungsspezifischer Korrekturfaktoren zum Bestimmen der Konzentration eines Analyten in einer Probe unter Verwendung der einen oder mehreren Messungen des ersten Signals und der einen oder mehreren Messungen des zweiten Signals umfasst.

2. Verfahren nach Anspruch 1, wobei die eine oder mehreren Messzellen frei von Analyten sind.

3. Verfahren nach einem der Ansprüche 1 bis 2, wobei das Verfahren nicht die Verwendung einer Analyt enthaltenden Probe mit einer bekannten Konzentration des Analyten einschließt.

4. Verfahren nach einem der Ansprüche 1 bis 3, wobei die Probe ein Körperfluid ist, vorzugsweise das Körperfluid Vollblut, Urin, Blutplasma oder Blutserum ist, insbesondere das Körperfluid Vollblut oder Blutplasma ist.

5. Verfahren nach einem der Ansprüche 1 bis 4, wobei der Analyt ausgewählt ist aus der Gruppe bestehend aus NT-proBNP (N-terminales Prohormon des Brain Natriuretic Peptide), Procalcitonin (PCT), BNP (Brain Natriuretic Peptide), D-Dimer, kardialem Troponin I (cTnI), kardialem Troponin T (cTnT), beta-hCG (humanes Choriongonadotropin), C-reaktivem Protein (CRP), Myoglobin und CK-MB (Kreatininkinase-Muskel/Gehirn).

6. Verfahren nach einem der Ansprüche 1 bis 5, wobei das erste Signal und das zweite Signal Fluoreszenzsignale sind.

7. Verfahren nach einem der Ansprüche 1 bis 6, wobei das erste Signal und das zweite Signal durch eine Photomultiplier-Röhre der Vorrichtung erfasst werden.

8. Verfahren nach einem der Ansprüche 1 bis 7, wobei Schritt b) einen Inkubationsschritt nach dem Hinzufügen des Testpuffers umfasst.

9. Verfahren nach Anspruch 6, wobei die Fluoreszenzsignale ein zeitaufgelöstes Fluoreszenzsignal sind.

10. Verfahren zum Bestimmen der Konzentration eines Analyten in einer Probe, wobei das Verfahren die Schritte

i) Kalibrieren einer Immunoassay-Vorrichtung gemäß dem Verfahren nach einem der Ansprüche 1 bis 9 unter Verwendung einer oder mehrerer Messzellen aus einer Charge identischer Messzellen, wobei jede der identischen Messzellen einen auf der Innenfläche der jeweiligen Messzelle immobilisierten Fänger-Antikörper und einen Tracer-Antikörper enthält, oder wobei jede der identischen Messzellen ein an der Innenfläche der jeweiligen Messzelle immobilisiertes Capture-Aptamer und ein Tracer-Aptamer enthält, wobei das Kalibrierverfahren dadurch ein oder mehrere vorrichtungsspezifische Korrekturfaktoren bereitstellt;
ii) Bereitstellen einer Probe, die den Analyten enthält;
iii) Inkontaktbringen der Probe mit einer Messzelle, ausgewählt aus der Charge identischer Messzellen;
iv) Waschen der Messzelle;
v) optionales Trocknen der Messzelle;
vi) Messen eines Probensignals aus der Messzelle unter Verwendung der Immunoassay-Vorrichtung; und
vii) Berechnen der Konzentration des Analyten aus dem Probensignal und mindestens dem einen oder den mehreren vorrichtungspezifischen Korrekturfaktoren umfasst.

11. Verfahren nach Anspruch 10, wobei die Berechnung der Konzentration des Analyten zusätzlich ein oder mehrere messzellenspezifische Korrekturfaktoren einschließt.

12. Verfahren nach einem der Ansprüche 1 bis 11, wobei zum Bestimmen der Konzentration einer Probe folgende Berechnung angewendet werden kann:

$$M = \alpha^* \ R(c) + b$$

wobei

- M ein gemessenes Signal ist,
- der Faktor $\alpha$ ein vorrichtungsspezifischer Sensitivitätskorrekturfaktor für die Vorrichtung ist, der aus dem ersten Signal aus der Messung in Schritt c) abgeleitet wird,
- R(c) eine von der spezifischen Vorrichtung unabhängige Reaktionsfunktion über der Konzentration ist, und

- das Additiv b das Hintergrundsignal für die Vorrichtung ist und aus dem in Schritt f) erhaltenen zweiten Signal abgeleitet werden kann.

13. Verfahren nach einem der Ansprüche 1 bis 11, wobei die Berechnung der vorrichtungsspezifischen Korrekturfaktoren eine Korrelation des gemessenen ersten und zweiten Signals der Vorrichtung mit einem jeweiligen ersten und zweiten Signal umfasst, das für eine oder mehrere unterschiedliche Vorrichtungen aus der gleichen Serie erhalten wurde, die zum Bestimmen von messzellenspezifischen Korrekturfaktoren verwendet wurden.

**Revendications**

1. Procédé d'étalonnage d'un dispositif d'immunoessai, le dispositif étant adapté pour déterminer la concentration d'un analyte dans un échantillon en mettant l'échantillon en contact avec une cellule de mesure sélectionnée parmi un lot de cellules de mesure identiques, dans lequel chacune des cellules de mesure identiques contient un anticorps de capture immobilisé sur la surface interne de la cellule de mesure respective et un anticorps traceur, ou dans lequel chacune des cellules de mesure identiques contient un aptamère de capture immobilisé sur la surface interne de la cellule de mesure respective et un aptamère de traceur, et mesurant un signal d'échantillon provenant de la cellule de mesure en utilisant le dispositif,
dans lequel le procédé comprend les étapes de

   a) la fourniture d'une ou plusieurs cellules de mesure à partir du lot de cellules de mesure identiques ;
   b) l'ajout de tampon de dosage aux une ou plusieurs cellules de mesure ;
   c) la mesure d'un premier signal pour chacune des une ou plusieurs cellules de mesure ;
   d) le lavage des une ou plusieurs cellules de mesure ;
   e) le séchage facultatif des une ou plusieurs cellules de mesure ;
   f) la mesure d'un second signal pour chacune des une ou plusieurs cellules de mesure ; et
   g) le calcul d'un ou plusieurs facteurs de correction spécifiques à un dispositif pour déterminer la concentration d'un analyte dans un échantillon en utilisant les une ou plusieurs mesures du premier signal et les une ou plusieurs mesures du second signal.

2. Procédé selon la revendication 1, dans lequel les une ou plusieurs cellules de mesure sont exemptes d'analyte.

3. Procédé selon l'une quelconque des revendications 1 et 2, dans lequel le procédé n'inclut pas l'utilisation d'un échantillon contenant un analyte présentant une concentration connue de l'analyte.

4. Procédé selon l'une quelconque des revendications 1 à 3, dans lequel l'échantillon est un fluide corporel, de préférence le fluide corporel est du sang total, de l'urine, du plasma sanguin ou du sérum sanguin, en particulier le fluide corporel est du sang total ou du plasma sanguin.

5. Procédé selon l'une quelconque des revendications 1 à 4, dans lequel l'analyte est choisi dans le groupe consistant en NT-proBNP (prohormone N-terminale du peptide natriurétique cérébral), procalcitonine (PCT), BNP (peptide natriurétique cérébral), D-dimère, troponine I cardiaque (cTnI), troponine T cardiaque (cTnT), bêta-hCG (gonadotrophine chorionique humaine), protéine C réactive (CRP), myoglobine et CK-MB (créatinine kinase-muscle/cerveau).

6. Procédé selon l'une quelconque des revendications 1 à 5, dans lequel le premier signal et le second signal sont des signaux de fluorescence.

7. Procédé selon l'une quelconque des revendications 1 à 6, dans lequel le premier signal et le second signal sont détectés par un tube photomultiplicateur du dispositif.

8. Procédé selon l'une quelconque des revendications 1 à 7, dans lequel l'étape b) comprend une étape d'incubation après l'ajout du tampon de dosage.

9. Procédé selon la revendication 6, dans lequel les signaux de fluorescence sont un signal de fluorescence à résolution temporelle.

10. Procédé de détermination de la concentration d'un analyte dans un échantillon, le procédé comprenant les étapes de

i) l'étalonnage d'un dispositif d'immunoessai selon le procédé de l'une quelconque des revendications 1 à 9 en utilisant une ou plusieurs cellules de mesure à partir d'un lot de cellules de mesure identiques, dans lequel chacune des cellules de mesure identiques contient un anticorps de capture immobilisé sur la surface interne de la cellule de mesure respective et un anticorps traceur, ou dans lequel chacune des cellules de mesure identiques contient un aptamère de capture immobilisé sur la surface interne de la cellule de mesure respective et un aptamère traceur, le procédé d'étalonnage fournissant ainsi un ou plusieurs facteurs de correction spécifiques au dispositif ;

ii) la fourniture d'un échantillon contenant l'analyte ;

iii) la mise en contact de l'échantillon avec une cellule de mesure choisie dans le lot de cellules de mesure identiques ;

iv) le lavage de la cellule de mesure ;

v) facultativement, le séchage de la cellule de mesure ;

vi) la mesure d'un signal d'échantillon provenant de la cellule de mesure en utilisant le dispositif d'immunoessai ; et

vii) le calcul de la concentration de l'analyte à partir du signal d'échantillon et au moins des un ou plusieurs facteurs de correction spécifiques au dispositif.

11. Procédé selon la revendication 10, dans lequel le calcul de la concentration de l'analyte inclut en outre un ou plusieurs facteurs de correction spécifiques à la cellule de mesure.

12. Procédé selon l'une quelconque des revendications 1 à 11, dans lequel le calcul suivant peut être appliqué pour déterminer la concentration d'un échantillon :

$$M = \alpha * R(c) + b$$

dans lequel

- M est un signal mesuré,
- le facteur $\alpha$ est un facteur de correction de sensibilité spécifique au dispositif pour le dispositif dérivé du premier signal de la mesure à l'étape c),
- R(c) est une fonction de réponse sur concentration qui est indépendante du dispositif spécifique, et
- l'additif b est le signal de fond pour le dispositif et est dérivable du second signal obtenu à l'étape f).

13. Procédé selon l'une quelconque des revendications 1 à 11, dans lequel le calcul des facteurs de correction spécifiques au dispositif comprend une corrélation des premier et second signaux mesurés du dispositif avec les premier et second signaux respectifs obtenus pour un ou plusieurs dispositifs différents de la même série qui ont été utilisés pour déterminer des facteurs de correction spécifiques à la cellule de mesure.

Figure 1

A

Ready-to-use    Incubation    Measurement    Washing    Measurement
                                            & drying

616 nm                                616 nm

B

Capture        Antigen in      Labeled tracer          Sandwitch         Unbound tracer
antibodies     blood sample    antibody                complex           antibody

Incubation

Figure 2

Figure 3

Figure 4

**Signal Level vs. Concentration Level**

Figure 5

**CorrectedCurrent vs. Concentration Level**

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0892927 B1
- US 3960497 A
- US 6277584 B
- WO 2014143323 A1
- US 9944657 B
- US 7922986 B2

### Non-patent literature cited in the description

- **KUNZ et al.** Addressing the challenges of biomarker calibration standards in ligand-binding assays: a European Bioanalysis Forum perspective. *Bioanalysis*, 2017, vol. 9 (19), 1493-1508
- Immunochemistry 1: a practical approach. Oxford: IRL Press, 1997, 193-214